# EUROPÄISCHE PATENTANMELDUNG

(11) **EP 4 686 754 A1**
(43) Veröffentlichungstag der Anmeldung: **04.02.2026**
(21) Anmeldenummer: 25170894.7
(22) Anmeldetag: 16.04.2025
(51) Int. Cl.: C12M 1/107, F17C 3/02, F17C 1/00

(54) **GASSPEICHER**

(30) Priorität: 31.07.2024 DE 102024121877
(71) Anmelder: JOPE Beteiligungs GmbH, 87653 Eggenthal (DE)
(72) Erfinder: Baur, Josef, 87653 Eggenthal (DE); Baur, Peter, 87653 Eggenthal (DE)
(74) Vertreter: Patentanwälte Olbricht, Buchhold, Keulertz Partnerschaft mbB

(57) **Zusammenfassung**

Ein Gasspeicher (10) hat eine Membran (20), die ein Gasvolumen (G) überspannt, und die mit einem Membranrand (22) über dem Gasvolumen (G) befestigt ist, und ein Halteelement (30), wobei der Membranrand (22) mit dem Haltelement (30) verbunden ist. Um die herkömmlichen Konstruktionen mit Betonfundament zu vereinfachen, sieht die Erfindung vor, dass das Halteelement (30) als Ballastelement ausgebildet ist und lösbar auf einem Aufnahmeelement (40) aufliegt. Das Halteelement (30) ist ferner ein Hohlkörper (34), der wenigstens eine Füllöffnung (36) für ein Ballastmaterial (32) aufweist, wobei das Haltelement (30) mit dem Ballastmaterial (32) befüllt oder befüllbar ist. Zweckmäßig ist der Hohlkörper (34) schlauchförmig ausgebildet und bei Bedarf in Kammern (38) unterteilt.

## Beschreibung

Die Erfindung betrifft einen Gasspeicher gemäß Anspruch 1.

Gasspeicher, insbesondere solche für Biogas, sind bekannt. Sie dienen der unmittelbaren Speicherung des teilweise direkt im Gasspeicher produzierten Gases. Dabei hat sich, ausgehend von den nicht konstanten Füllvolumina, ein flexibles Membrandach als Stand der Technik etabliert.

Zur Befestigung und zum gasdichten Abschluss des Membrandachs wird die Membran an ihren Enden meist auf einem Betonfundament verankert.

Sowohl beim Aufbau als auch beim Abbruch oder einem evtl. nötigen Tausch der Membran sind daher umfangreiche Montage- und Bauarbeiten notwendig.

Die Erfindung hat die entsprechende Verbesserung des Stands der Technik zur Aufgabe.

Die Aufgabe ist erfindungsgemäß gelöst mit einem Gasspeicher gemäß Anspruch 1.

Ausgestaltungen sind Gegenstand der Unteransprüche und der Ausführungsformen.

Erfindungsgemäß wird ein Gasspeicher vorgeschlagen umfassend eine Membran, die ein Gasvolumen überspannt, und die mit einem Membranrand über dem Gasvolumen befestigt ist, und ein Halteelement, wobei der Membranrand mit dem Haltelement verbunden ist, dadurch gekennzeichnet, dass das Halteelement als Ballastelement ausgebildet ist und lösbar auf einem Aufnahmeelement aufliegt.

Aufgrund der Ausführung des Halteelements als Ballastelement entfällt die Notwendigkeit der baulichen Verankerung der Membran in einem Betonelement. Ferner erlaubt die lösbar ausgeführte Auflage auf dem Aufnahmeelement einen schnellen sowie kostengünstigen Aufbaus des Gasspeichers, da üblicherweise anfallende Arbeiten wie Aushub-, Betontransport- und Betonarbeiten komplett entfallen. Ebenso entfallen Betonzerlegung und Abtransport beim Abbruch oder bei Demontagearbeiten im Falle eines evtl. notwendigen Wechsels der Membran bei Beschädigung.

Die Verbindung der Membran über den Membranrand mit dem Haltelement sorgt für einen direkten Lastübertrag auf das Haltelement, womit sich ein weiterer Vorteil der lösbaren Auflage des Ballastelementes auf dem Aufnahmeelement ergibt. Dieser besteht darin, dass das dynamische Verhalten eines Gasspeichers mit wechselnden Speichervolumina nicht zu Schäden an einer Unterkonstruktion, wie im Stand der Technik definiert, führen kann und somit mit geringeren Reserven für die Lastannahme gerechnet werden kann.

Eine Weiterbildung sieht vor, dass das Haltelement mit einem Ballastmaterial befüllt oder befüllbar ist. Eine Befüllung des Halteelementes mit einem Ballastmaterial erlaubt eine, auf die entsprechende Situation abgestimmte, individuelle Befüllung

Gemäß einer anderen Weiterbildung ist das Halteelement ein Hohlkörper, der wenigstens eine Füllöffnung für das Ballastmaterial aufweist. Eine Füllöffnung erlaubt ein späteres Füllen an bspw. dem vorgesehenen Ort des Gasspeichers womit vermieden wird, dass schwere Lasten über weite Strecken bewegt werden müssen.

Gemäß einer weiteren Weiterbildung ist der Hohlkörper schlauchförmig ausgebildet. Schlauchförmige Ausführungen erlauben eine individuelle Anpassung an die vor Ort herrschenden Gegebenheiten, womit auch evtl. vorhandene Unregelmäßigkeiten im Aufnahmeelement toleriert werden können.

In einer weiteren Variante ist der Hohlkörper in Kammern unterteilt ist. Die Unterteilung des Hohlkörpers in einzelne Kammern dient der Sicherheit, da bei Beschädigungen an einer Stelle des Hohlkörpers das Ballastmaterial in anderen Kammern des Hohlkörpers wenig beeinflusst wird. Zudem erlaubt die Verwendung mehrerer Kammern einen Ausgleich von Witterungsbedingungen indem bspw. luftgefüllte Kammern ein Ausgleichsvolumen bilden.

Typischerweise ist jeder Kammer des Hohlkörpers wenigstens eine Füllöffnung zugewiesen. Die Zuweisung mindestens einer Füllöffnung pro Kammer erlaubt die komplette Abschottung der Kammern untereinander und verhindert somit im Falle einer Beschädigung einer Kammer den kompletten Verlust des Ballastmaterials.

Vorzugsweise ist das Ballastmaterial ein fließfähiges Material, z.B. Wasser, Sand, Öl, Gülle, u. dgl.. Fließfähige Materialien sind besonders bei der Füllung unregelmäßig ausgebildeter Objekte, wie es bei Schläuchen der Fall sein kann, zu bevorzugen.

Typischerweise wird das Aufnahmeelement von einem Umgebungsbereich des Gasspeichers gebildet, oder das Aufnahmeelement ist ein eigenständiges Bauelement. Besonders die direkte Verwendung der Umgebung als Aufnahmeelement ist eine zu bevorzugende Lösung, da die Aspekte der Zeit- und Kostenersparnis dem Geist der Erfindung entsprechen.

In einer weiteren Ausführung weist das Aufnahmeelement eine Fläche auf, auf der das Haltelement aufliegt.

In einer Variante ist die Fläche im Wesentlichen eben, V-förmig, trapezförmig oder gerundet ausgebildet. Neben einer einfachen Auflagefläche ist sind insbesondere jene Ausführungen zu bevorzugen in denen das Aufnahmeelement aufgrund seiner Geometrie eine räumliche Beschränkung für das Halteelement bildet.

Typischerweise umgibt das Aufnahmeelement einen Bodenabschnitt, einen Reaktionsraum, ein Becken und/oder eine Lagune. Da Biogas oft am Ort der Speicherung direkt produziert wird ist es besonders vorteilhaft, wenn das Aufnahmeelement direkt um eine solche Produktionsstätte herum angeordnet ist.

In einer Weiterbildung ist der Bodenabschnitt, der Reaktionsraum, das Becken und/oder die Lagune mit einer gas- und/oder fluiddichten Basisfolie ausgelegt.

In einer anderen Variante überdeckt die Basisfolie das Aufnahmeelement zumindest abschnittsweise.

In einer besonders vorteilhaften Ausbildung überdeckt die Basisfolie die Fläche des Aufnahmeelements zumindest abschnittsweise. Um einen ungewollten Eintrag von im Gasspeicher befindlicher Produkte zu verhindern ist es vorteilhaft den Bodenabschnitt, den Reaktionsraum, das Becken und/oder die Lagune mit einer gas- und fluiddichten Basisfolie auszulegen.

In einer weiteren vorteilhaften Ausführung ist das Halteelement auf der Fläche des Aufnahmeelements beweglich angeordnet. Eine bewegliche Anordnung sorgt dafür, dass entstehende mechanische Belastungen des Gasspeichers, sei es durch Windlast und/oder Gasdruck, dynamisch aufgenommen werden können.

In einer besonders vorteilhaften Ausführung kann insbesondere durch das Bewegen des Halteelements im Aufnahmeelement das Volumen des Gasspeichers vergrößert werden. Ein Überdruck kann durch Ausnehmungen in dem Aufnahmeelement vermieden werden, indem die dichtende Auflage unterbrochen wird.

In einer weiteren Ausführung liegt das Halteelement stets sicher dichtend oder abgedichtet auf der Fläche des Aufnahmeelements und/oder der Basisfolie auf. Die dichtende Eigenschaft von Halteelement mit Aufnahmeelement und/oder Basisfolie findet durch einen Kraft- und/oder Formschluss statt.

Besonders vorteilhaft kann es sein, das Aufnahmeelement mit Wasser zu befüllen, um eine zusätzliche Dichtwirkung mit dem Halteelement zu erzielen.

Gemäß einer anderen Weiterbildung ist der Membranrand gasdicht mit dem Haltelement verbunden.

Gemäß einer anderen Ausführung sind der Membranrand und das Haltelement über ein Fixierelement miteinander verbunden.

Vorzugsweise ist das Fixierelement am Membranrand und/oder am Haltelement ausgebildet.

Insbesondere ist das Fixierelement zumindest abschnittsweise elastisch ausgebildet. Eine elastische Verbindung erlaubt es die aufzunehmenden Lasten dynamisch zu verteilen und punktuelle Überlastungen zu vermeiden.

Gemäß einer Weiterbildung bildet bzw. weist die Membran eine Wetterschutzfolie auf, wobei die Wetterschutzfolie mit der Membran und/oder dem Halteelement verbunden ist. Um die Standzeit der Membran zu erhöhen ist es vorteilhaft diese vor witterungsbedingten Einflüssen abzuschirmen.

Gemäß einer anderen Weiterbildung bildet bzw. weist die Membran eine Dichtfolie auf, wobei die Dichtfolie mit der Membran und/oder dem Halteelement verbunden ist.

Vorzugsweise ist zwischen der Wetterschutzfolie und der Dichtfolie ein Stützluftraum ausgebildet.

In einer anderen Variante ist zwischen der Basisfolie und der Dichtfolie ein Stützluftraum ausgebildet.

Ein Stützluftraum dient zum Stabilisieren einer Membran bestehend aus zwei oder mehr Folien. Während die gasumschließende Folie von diesem gestützt wird, sorgt die Stützluft dafür, dass die andere(n) Folie(n) nicht aufliegen. Dadurch ist außerdem eine klar definierte Form der Außenhülle sichergestellt. Bei der Speicherung leichter Gase, insbesondere solcher die leichter als die Umgebungsluft sind, sorgt die Stützluft zudem dafür, dass die Membran das Gasvolumen wohldefiniert begrenzt und so eine Volumenmessung möglich ist.

Gemäß einer weiteren Weiterbildung ist die Membran ein- oder mehrlagig ausgebildet.

Weitere Merkmale, Einzelheiten und Vorteile der Erfindung ergeben sich aus dem Wortlaut der Ansprüche sowie aus der folgenden Beschreibung von Ausführungsbeispielen anhand der Zeichnungen. Es zeigen:
- Fig. 1: eine perspektivische Ansicht eines Gasspeichers,
- Fig. 2: eine vergrößerte Ansicht des Haltelementes mit daran befestigter Membran,
- Fig. 3: eine detaillierte Ansicht des Haltelementes in einer typischen Ausführung,
- Fig. 4: schematische Darstellung des Gasspeichers bei der Speicherung eines schweren Gases in einer typischen Ausführung,
In den Figuren sind gleiche oder einander entsprechende Elemente jeweils mit den gleichen Bezugszeichen bezeichnet und werden daher, sofern nicht zweckmäßig, nicht erneut beschrieben. Bereits beschriebene Merkmale werden zur Vermeidung von Wiederholungen nicht erneut beschrieben und sind auf alle Elemente mit gleichen oder einander entsprechenden Bezugszeichen anwendbar, sofern nicht explizit ausgeschlossen. Die in der gesamten Beschreibung enthaltenen Offenbarungen sind sinngemäß auf gleiche Teile mit gleichen Bezugszeichen bzw. gleichen Bauteilbezeichnungen übertragbar. Auch sind die in der Beschreibung gewählten Lageangaben, wie z. B. oben, unten, seitlich usw. auf die unmittelbar beschriebene sowie dargestellte Figur bezogen und sind bei einer Lageänderung sinngemäß auf die neue Lage zu übertragen. Weiterhin können auch Einzelmerkmale oder Merkmalskombinationen aus den gezeigten und beschriebenen unterschiedlichen Ausführungsbeispielen für sich eigenständige, erfinderische oder erfindungsgemäße Lösungen darstellen.

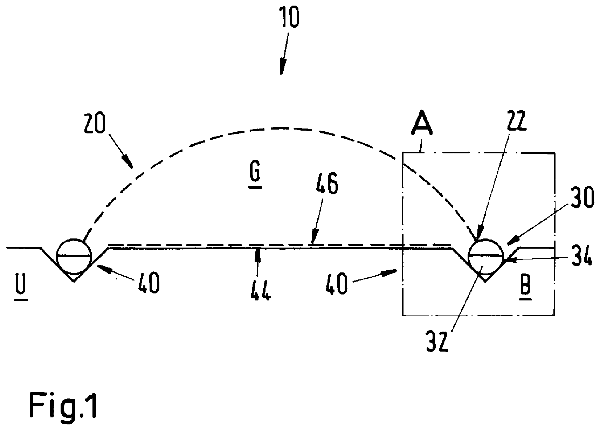

Die Figur 1 zeigt eine perspektivische Ansicht eines Gasspeichers 10. In der Umgebung U des Gasspeichers 10 ist ein Aufnahmeelement 40 ausgebildet in denen sich das Haltelement 30 lösbar befindet. Das Aufnahmeelement 40 kann direkt in der Umgebung U ausgebildet sein oder ein separates Bauelement B sein. Das Halteelement 30 weist einen Hohlkörper 34 auf der mit einem Ballastmaterial 32 gefüllt ist. Eine Membran 20 ist über einen Membranrand 22 mit dem Haltelement 30 verbunden und überspannt so ein Gasvolumen G. Der Bereich zwischen den Halteelementen 30 ist ein Bodenabschnitt 44 (bzw. ein Reaktionsraum, Becken oder Lagune) welcher von einer Basisfolie 44 bedeckt ist.

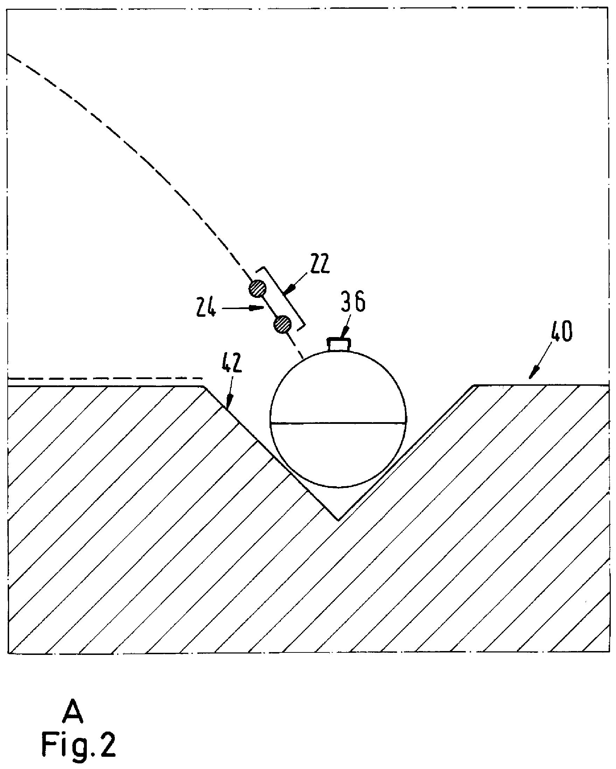

Die Figur 2 zeigt die detaillierte Darstellung des Haltelements 30 in dem Aufnahmeelement 40. Das Halteelement 30, welches hier als Schlauch ausgeführt ist, liegt lösbar auf einer Fläche 42 auf. Über eine Füllöffnung 36 kann Ballastmaterial in den Schlauch gefüllt werden. An dem Schlauch ist über ein Fixierelement 24 die Membran am Membranrand 22 verbunden.

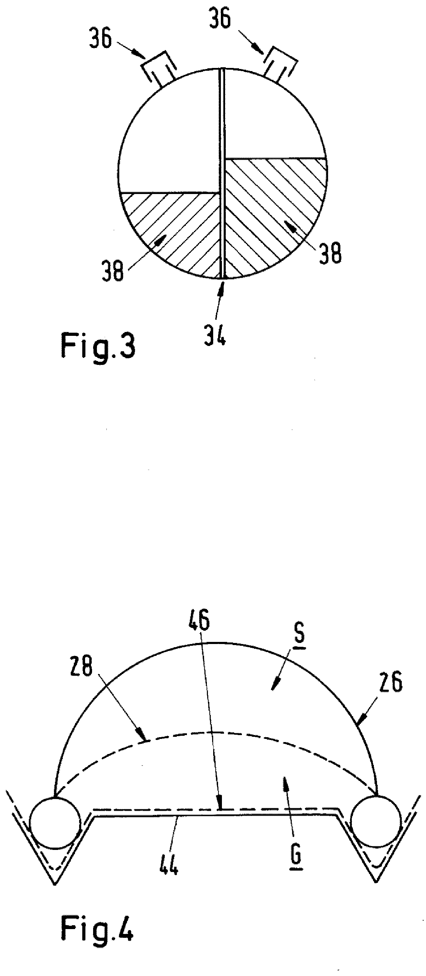

Figur 3 zeigt eine Ausführung des Haltelements als Hohlkörper 34 mit mehreren Kammern 38. Jede dieser Kammern weist eine Füllöffnung 36 auf über die das Ballastmaterial zugegeben werden kann.

Die Figur 4 zeigt den Gasspeicher in einer typischen Ausführung bei der Speicherung schwerer Gase. Die Basisfolie 46 erstreckt sich über den Bodenabschnitt 44 und ist auch in den Aufnahmeelementen, insbesondere der Fläche, ausgebildet. Darauf beweglich ausgebildet ist das Halteelement, an welchem eine Wetterschutzfolie 26 und eine Dichtfolie 28 ausgebildet sind. Im Falle schwerer Gase überspannt die Dichtfolie 28 das Gasvolumen und der Stützluftraum S ist zwischen Wetterschutzfolie 26 und Dichtfolie 28 ausgebildet, wodurch die äußere Form der Membran konstant bleibt.

Die Erfindung ist nicht auf eine der vorbeschriebenen Ausführungsformen beschränkt, sondern in vielfältiger Weise abwandelbar.

Man erkennt, dass ein erfindungsgemäßer Gasspeicher 10 eine Membran 20 hat, die ein Gasvolumen G überspannt, und die mit einem Membranrand 22 über dem Gasvolumen G befestigt ist. Der Gasspeicher hat zudem ein Halteelement 30, wobei der Membranrand 22 mit dem Haltelement 30 verbunden ist. Um herkömmliche Konstruktionen mit Betonfundament zu vereinfachen und um sowohl den Montageaufwand und die Kosten zu senken, sieht die Erfindung vor, dass das Halteelement 30 als Ballastelement ausgebildet ist und lösbar auf einem Aufnahmeelement 40 aufliegt. Das Halteelement 30 ist ferner ein Hohlkörper 34, der wenigstens eine Füllöffnung 36 für ein Ballastmaterial 32 aufweist, wobei das Haltelement 30 mit dem Ballastmaterial 32 befüllt oder befüllbar ist. Zweckmäßig ist der Hohlkörper 34 schlauchförmig ausgebildet und bei Bedarf in Kammern 38 unterteilt.

Mit dieser Konstruktion werden Kräfte, die vom Gasspeicher über den Gasdruck oder über Witterungseinflüsse, z.B. Windlasten, erzeugt werden, von dem mit einer Flüssigkeit gefüllten Halteelemente, vorzugsweise ein Schlauch, gehalten.

Der Schlauch kann in einem Werk vorgefertigt und konfektioniert werden.

Vor Ort auf der Baustelle wird der Schlauch sodann mit dem Ballastelemente, vorzugsweise einer Flüssigkeit, gefüllt. Hierbei kann beispielsweise unbehandeltes Wasser verwendet werden, das meist vor Ort und damit ohne großen Transportaufwand verfügbar ist.

Die Membran und die darauf einwirkenden Kräfte werden von der Schwerkraft, die auf den gefüllten Schlauch wirkt, gehalten.

Das je nach Größe, Ausführung und Betriebsparametern des Gasspeichers erforderliche Gewicht des Haltelements kann über einen entsprechenden Schlauchdurchmesser und die dadurch einhergehende Volumenänderung eingestellt werden.

Der Schlauch kann sowohl im Querschnitt als auch am Speicher längslaufend mit mehreren Kammern ausgerüstet werden, um einen eventuellen Schaden lokal zu begrenzen.

Der Schlauch kann auch mit einer luft- oder gasgefüllten Kammer als Ausgleichsvolumen bei Eisbildung oder für Temperaturbedingte Volumenänderungen versehen werden.

Der Schlauch wird zur Lagesicherung und zur Abdichtung des Gasraumes in einem V- oder Trapezförmigen Graben positioniert.

Die erfindungsgemäße Konstruktion kann auch mit deutlich geringeren Kräften bei der Lastannahme gerechnet werden, weil eine Überlastung durch das dynamische und Verhalten beim Betrieb des Gasspeichers nicht zu Schäden an der Konstruktion führen kann.

Sämtliche aus den Ansprüchen, der Beschreibung und der Zeichnung hervorgehenden Merkmale und Vorteile, einschließlich konstruktiver Einzelheiten, räumlicher Anordnungen und Verfahrensschritten, können sowohl für sich als auch in den verschiedensten Kombinationen erfindungswesentlich sein.

In den Rahmen der Erfindung fallen sämtliche Kombinationen aus zumindest zwei von den in der Beschreibung, den Ansprüchen und/oder den Figuren offenbarten Merkmalen.

Zur Vermeidung von Wiederholungen sollen vorrichtungsgemäß offenbarte Merkmale auch als verfahrensgemäß offenbart gelten und beanspruchbar sein. Ebenso sollen verfahrensgemäß offenbarte Merkmale als vorrichtungsgemäß offenbart gelten und beanspruchbar sein.

### Bezugszeichenliste

- 10: Gasspeicher
- 20: Membran
- 22: Membranrand
- 24: Fixierelement
- 26: Wetterschutzfolie
- 28: Dichtfolie
- 30: Halteelement
- 32: Ballastmaterial
- 34: Hohlkörper
- 36: Füllöffnung
- 38: Kammer
- 40: Aufnahmeelement
- 42: Fläche
- 44: Bodenabschnitt/Reaktionsraum/Becken/Lagune
- 46: Basisfolie
- B: Bauelement
- U: Umgebungsbereich
- S: Stützluftraum
- G: Gasraum

## Patentansprüche

1. Gasspeicher (10), umfassend
▪ eine Membran (20), die ein Gasvolumen (G) überspannt, und die mit einem Membranrand (22) über dem Gasvolumen (G) befestigt ist, und
▪ ein Halteelement (30), wobei der Membranrand (22) mit dem Haltelement (30) verbunden ist,
**dadurch gekennzeichnet, dass**
das Halteelement (30) als Ballastelement ausgebildet ist und lösbar auf einem Aufnahmeelement (40) aufliegt.

2. Gasspeicher (10) gemäß Anspruch 1, **dadurch gekennzeichnet, dass** das Haltelement (30) mit einem Ballastmaterial (32) befüllt oder befüllbar ist, wobei das Halteelement (30) ein Hohlkörper (34) ist, der wenigstens eine Füllöffnung (36) für das Ballastmaterial (32) aufweist.

3. Gasspeicher (10) gemäß Anspruch 3, **dadurch gekennzeichnet, dass** der Hohlkörper (34) schlauchförmig ausgebildet ist.

4. Gasspeicher (10) gemäß Anspruch 3 oder 4, **dadurch gekennzeichnet, dass** der Hohlkörper (34) in Kammern (38) unterteilt ist, wobei jeder Kammer (38) des Hohlkörpers (34) wenigstens eine Füllöffnung (36) zugewiesen ist.

5. Gasspeicher (10) gemäß einem der Ansprüche 2 bis 6, **dadurch gekennzeichnet, dass** das Ballastmaterial (32) ein fließfähiges Material ist, z.B. Wasser, Sand, Öl, u. dgl..

6. Gasspeicher (10) gemäß einem der vorherigen Ansprüche, **dadurch gekennzeichnet, dass** das Aufnahmeelement (40) von einem Umgebungsbereich (U) des Gasspeichers (10) gebildet ist, oder dass das Aufnahmeelement (40) ein eigenständiges Bauelement (B) ist.

7. Gasspeicher (10) gemäß einem der vorherigen Ansprüche, **dadurch gekennzeichnet, dass** das Aufnahmeelement (40) eine Fläche (42) aufweist, auf der das Haltelement (40) aufliegt, wobei die Fläche (42) im Wesentlichen eben, V-förmig, trapezförmig oder gerundet ausgebildet ist.

8. Gasspeicher (10) gemäß Anspruch 8 bis 10, **dadurch gekennzeichnet, dass** das Aufnahmeelement (40) einen Bodenabschnitt (44), einen Reaktionsraum (44), ein Becken (44) und/oder eine Lagune (44) umgibt.

9. Gasspeicher (10) gemäß Anspruch 11, **dadurch gekennzeichnet, dass** der Bodenabschnitt (44), der Reaktionsraum (44), das Becken (44) und/oder die Lagune (44) mit einer gas- und/oder fluiddichten Basisfolie (46) ausgelegt ist.

10. Gasspeicher (10) gemäß einem der vorherigen Ansprüche, **dadurch gekennzeichnet, dass** das Halteelement (30) auf der Fläche des Aufnahmeelements (40) beweglich angeordnet ist.

11. Gasspeicher (10) gemäß einem der vorherigen Ansprüche, **dadurch gekennzeichnet, dass** das Halteelement (30) dichtend oder abgedichtet auf der Fläche (42) des Aufnahmeelements (40) und/oder der Basisfolie (46) aufliegt.

12. Gasspeicher (10) gemäß einem der vorherigen Ansprüche, **dadurch gekennzeichnet, dass** der Membranrand (22) gasdicht mit dem Haltelement (30) verbunden ist, wobei der Membranrand (22) und das Haltelement (30) über ein Fixierelement (24) miteinander verbunden sind.

13. Gasspeicher gemäß Anspruch 17 oder 18, **dadurch gekennzeichnet, dass** das Fixierelement (24) am Membranrand (22) und/oder am Haltelement (40) ausgebildet ist.

14. Gasspeicher (10) gemäß einem der vorherigen Ansprüche, **dadurch gekennzeichnet, dass** die Membran (20) eine Wetterschutzfolie (26) bildet oder aufweist, wobei die Wetterschutzfolie (26) mit der Membran (20) und/oder dem Halteelement (40) verbunden ist.

15. Gasspeicher (10) gemäß einem der vorherigen Ansprüche, **dadurch gekennzeichnet, dass** die Membran (20) eine Dichtfolie (28) bildet oder aufweist, wobei die Dichtfolie (28) mit der Membran (20) und/oder dem Halteelement (40) verbunden ist.
